Europäisches Patentamt

European Patent Office

Office européen des brevets

(11) Publication number: **0 405 015**
**A1**

# EUROPEAN PATENT APPLICATION

(21) Application number: 89201693.2

(51) Int. Cl.⁵: **C07C 303/42, C07C 309/30**

(22) Date of filing: **27.06.89**

The title of the invention has been amended (Guidelines for Examination in the EPO, A-III, 7.3).

(43) Date of publication of application:
**02.01.91 Bulletin 91/01**

(84) Designated Contracting States:
**AT BE CH DE ES FR GB GR IT LI LU NL SE**

(71) Applicant: **BPCO INC.**
**0244 rue St. Patrick**
**La Salle, PQ H8R 1R8(CA)**

(72) Inventor: **Vines, Solomon Martin**
**54, Ainslie Road**
**Montreal Quebec H4X 1K6(CA)**
Inventor: **Cote, Jacynthe**
**2410 Le Dieppe**
**Jonquieres, PQ(CA)**

(74) Representative: **Smulders, Theodorus A.H.J., Ir. et al**
**Vereenigde Octrooibureaux Nieuwe Parklaan 107**
**NL-2587 BP 's-Gravenhage(NL)**

(54) Reduction of the tendency of strong acids to form solid hydrates.

(57) A method of preventing crystallization by reducing the water sensitivity of a strong acid, such as p-toluene sulfonic acid, which comprises mixing therewith an effective amount of a polar aprotic solvent such as DMF or DMSO. By this method, the water sensitivity is reduced to levels whereby exposure of the acid to the atmosphere is possible without formation of crystals.

EP 0 405 015 A1

# REDUCING SENSITIVITY OF STRONG ACIDS

This invention relates to the stabilization of strong acids upon exposure to moisture by reduction of their water sensitivity.

Strong acids are used as catalysts in many industrial processes, including polymerisation. Typical of such catalysts are toluene sulfonic acids, as described in Mausner et al, U.S. Patent no. 3,458,449. This patent teaches the production of improved toluene sulfonic acid compositions as substantially anhydrous liquid mixtures. However, it has been found that these materials are extremely sensitive to moisture and within a short period (often less than one hour) of exposure to the atmosphere, crystals form. These precipitated crystals cause a great deal of inconvenience and handling difficulties in industrial processes as they tend to clog lines, filters, etc.

It has been attempted to alleviate this problem by use of polar solvents, such as alcohols, but such approach has only been partially effective. When alcohols such as methanol, ethanol, isopropanol and ethylene glycol are added to a strong acid catalyst and the mixture exposed to moist air, crystals typically form in a relatively short time which represents only a marginal improvement over the crystallization period for the untreated acid.

Therefore, the object of the present invention is to provide a means of reducing the water sensitivity of strong acids to levels whereby exposure to the atmosphere is possible without the crystallization problems normally associated with such materials.

In accordance with the present invention, we have found that, unexpectedly, whilst polar solvents such as alcohols are relatively ineffective to reduce the water sensitivity of strong acids such as p-toluene sulfonic acids, polar aprotic solvents such as N,N-diethylformamide; dimethyl sulfoxide; N,N-dimethylformamide; hexamethylphosphoramide; dimethylpropylene urea sulpholane; and N-methyl-2-pyrrolidone are highly effective for this purpose. Whilst we do not wish to be bound by theory, it is believed that such solvents might be effective through suppression of the formation of insoluble hydrates or by solubilising the hydrates once formed. Also, it is believed that the polar aprotic solvents do not offer potential reaction sites at the low pH of the acids, which would explain the much more stable mixtures, especially at the elevated temperatures often used for reactions involving these strong acids as catalysts.

The invention will now be described further by reference to the following examples.

## EXAMPLE 1

200 g. of Witco "Ultra T-X" (trademark for a p-toluenesulfonic acid catalyst from Witco Chemical Corporation) in a 500 ml beaker was exposed to air at 25°C, 50% relative humidity. Within a few minutes, crystals appeared in the solution and within 4 to 5 hours, the material became a solid mass. In a second experiment using the same catalyst under the same conditions, a drop of water was added and precipitation was immediate.

## EXAMPLE 2

The experiment of Example 1 was repeated with the addition of methanol, ethanol, isopropanol and ethylene glycol, respectively, over a wide range of concentrations. Crystals began to form within an hour or less.

## EXAMPLE 3

The "Ultra T-X" was firstly treated by addition of 10% N,N-dimethylformamide (DMF) in an amount of 10% by volume of the total mixture and the experiment then followed the procedure of Example 1. Crystals appeared within an hour - considerably more slowly than was the case with the untreated acid and comparable to the best results obtained with the alcohol-treated acid.

## EXAMPLE 4

The experiment of Example 3 was repeated with varying amounts of DMF (20%; 30%; 40% ... up to 65%) by volume of the total mixture and precipitation was retarded for up to 36 hours from initial exposure to the atmosphere. For additions of 30% DMF and greater, up to 200% v/v water could be added before the solution became cloudy, denoting precipitation.

## EXAMPLE 5

The experiment of Example 4 was repeated using dimethyl sulfoxide (DMSO) as additive in an amount of 20% by volume, instead of DMF. The solution was found to be stable for up to 36 hours exposure to the atmosphere and, again, 200% v/v water could be added before precipitation.

Although the foregoing examples illustrate the invention by reference to the use of DMF and DMSO, higher homologues, such as N,N-diethylformamide and other polar aprotic solvents such as those listed above are effective in the suppression of water sensitivity of strong acids.

## Claims

1. A method of reducing the water sensitivity of a strong acid which comprises mixing therewith after production of said acid, an effective amount of a polar aprotic solvent in an amount effective to inhibit crystal formation upon exposure of said acid to moisture.

2. The method of claim 1, wherein said polar aprotic solvent is selected from N,N-dimethylformamide; N,N-diethylformamide; dimethyl sulfoxide; hexamethyl phosphoramide; dimethylpropylene urea; sulpholane; and N-methyl-2-pyrrolidone.

3. The method of claim 2, wherein said polar aprotic solvent is N,N-dimethylformamide.

4. The method of claim 2, wherein said polar aprotic solvent is dimethyl sulfoxide.

5. The method of claim 2, wherein said strong acid is p-toluene sulfonic acid.

6. The method of claim 2, wherein the amount of said polar aprotic solvent added is at least about 10% by volume of the total mixture.

7. The method of claim 2, wherein the amount of said polar aprotic solvent added is at least about 20% by volume of the total mixture.

8. The method of claim 2, wherein said acid is p-toluene sulfonic acid and said polar aprotic solvent is selected from N,N-dimethylformamide and dimethyl sulfoxide.

9. The method of claim 8, wherein said polar aprotic solvent is added in an amount of at least about 10% by volume of the total mixture.

10. The method of claim 9, wherein said polar aprotic solvent is added in an amount of from about 20% to about 65% by volume of the total mixture.

11. A composition comprising a strong acid in admixture with a polar aprotic solvent in an amount effective to inhibit crystal formation upon exposure of said acid to moisture thereby to impart reduced water sensitivity to said acid.

12. The composition of claim 11, wherein said polar aprotic solvent is selected from N,N-dimethylformamide; N,N-diethylformamide; dimethyl sulfoxide; hexamethyl phosphoramide; dimethylpropylene urea; sulpholane; and N-methyl-2-pyrrolidone.

13. The composition of claim 12, wherein said polar aprotic solvent is N,N-dimethylformamide.

14. The composition of claim 12, wherein said polar aprotic solvent is dimethyl sulfoxide.

15. The composition of claim 12, wherein said strong acid is p-toluene sulfonic acid.

16. The composition of claim 12, wherein the amount of said polar aprotic solvent is at least about 10% by volume of the total mixture.

17. The composition of claim 12, 13 or 14, wherein the amount of said polar aprotic solvent is at least about 20% by volume of the total mixture.

18. The composition of claim 12, wherein said acid is p-toluene sulfonic acid and said polar aprotic solvent is selected from N,N-dimethylformamide and dimethyl sulfoxide.

19. The composition of claim 18, wherein said polar aprotic solvent is present in an amount of at least about 10% by volume of the total mixture.

20. The composition of claim 19, wherein said polar aprotic solvent is present in an amount of from about 20% to about 65% by volume of the total mixture.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-3 458 449 (M. MAUSNER)<br>* abstract; claim 1 *<br>--- | 1 | C 07 C 303/42<br>C 07 C 309/30 |
| A | FR-A-2 291 267 (PROCTER & GAMBLE)<br>* claim 1 *<br>--- | 1 | |
| A | US-A-2 807 641 (D. W. MILNER et al.)<br>* column 3, lines 12-17 *<br>--- | 1 | |
| A | US-A-3 634 271 (C.A. FRIEDMAN)<br>* column 1, line 21 - column 2, line 22 * | 1 | |

TECHNICAL FIELDS SEARCHED (Int. Cl.5)

C 07 C 303/00
C 07 C 309/00

*The present search report has been drawn up for all claims*

| Place of search | Date of completion of the search | Examiner |
|---|---|---|
| BERLIN | 13-02-1990 | PROBERT C.L. |